# EUROPÄISCHE PATENTSCHRIFT

(11) **EP 1 046 405 B1**
(45) Veröffentlichungstag und Bekanntmachung des Hinweises auf die Patenterteilung: **23.06.2004**
(21) Anmeldenummer: 00108439.1
(22) Anmeldetag: 18.04.2000
(51) Int. Cl.: A61M 15/02, A61M 15/00

(54) **Aerosolvernebler**
Aerosol nebulizer
Nébuliseur d'aérosol

(30) Priorität: 24.04.1999 DE 29907307 U
(43) Veröffentlichungstag der Anmeldung: 25.10.2000
(73) Patentinhaber: Büttner, Klaus, D-25336 Klein Nordende (DE)
(72) Erfinder: Büttner, Klaus, D-25336 Klein Nordende (DE)
(74) Vertreter: DIEHL GLAESER HILTL & PARTNER

(56) Entgegenhaltungen:
- EP-A- 0 590 752
- WO-A-93/17741
- WO-A-97/07896
- GB-A- 841 147
- US-A- 4 185 316
- US-A- 5 267 555

## Beschreibung

Die Erfindung bezieht sich auf eine Vorrichtung mit einem Ultraschallvernebler, der ein Sauerstoff mitführendes Aerosol für therapeutische Zwecke erzeugt, welches entweder aus reinem Wasser, einem Wassergemisch mit mindestens einem therapeutischen Wirkstoff oder lediglich aus einem therapeutischen Wirkstoff gebildet wird.

Eine derartige Vorrichtung mit einem Ultraschallvernebler ist bekannt (GB 841 147 A) und dient der Erzeugung eines Luftstromes, mit dessen Hilfe Arzneimittel über Inhalation verabreicht werden können.

Die Aerosol- bzw. Inhalationstherapie, bei welcher gelöste, zu Nebel zerstäubte Medikamente (Teilchengröße. <10µm) vom Patienten eingeatmet werden, ist eine etablierte Methode zur Darreichung von Medikamenten, z. B. bei der Behandlung von obstruktiven Atemwegserkrankungen, Bronchiektasen, zystischer Fibrose. Bei den eingesetzten Medikamenten handelt es sich z. B. um Beta-2-Sympatomimetika, Anticholinergika, Kortikosteroide, Cromoglicin-säure (DNCG), Pentamidin und dergleichen.

Bei bekannten Vorrichtungen der eingangs genannten Art erzeugt der Ultraschallvernebler das therapeutisch eingesetzte Aerosol und allein durch Einatmen dieses Aerosols kann erreicht werden, dass das Medikament in die Lunge, und zwar dort in die Alveolen gelangt. Dies bedeutet, dass das Medikament fein zerstäubt eine lokal außerordentliche gute Wirkung erzeugt und unter anderem auch in den Bronchien festgesetztes Sekret gelöst werden kann. Wenngleich in diesem Zusammenhang über Erfolge berichtet wird, so hat aber auch dieses therapeutische Verfahren Grenzen, und es ist erkannt worden, dass im Aerosol mitgeführter Sauerstoff entweder nicht in genügender Menge und/oder nicht in der optimalen Konzentration im Hinblick auf das Arzneimittel und auch nicht im Hinblick auf die Esscheinungsform des Sauerstoffs optimal wirken kann.

Es ist ein System zur Erzeugung einer Mixtur bekannt (WO-A-93 17741), die Stick oxyde und Luft zur medizinischen

Anwendung erzeugt. Der Luftstrom, der die entsprechenden Wirkstoffpartikel mit sich führt, wird durch einen elektrischen Lichtbogen hindurchgeführt, welcher durch Hochspannungselektroden erzeugt wird. Auf diese Art und Weise wird Stickoxyd mit Luft gemischt und diese Mixtur wird insgesamt als therapeutischer Wirkstoff dem Patienten verabreicht.

Es ist weiterhin ein Verfahren zum Verabreichen eines ionisierten Aerosols mit Medizinteilchen bekannt (US-A-5267555). Bei diesem Verfahren wird ein ionisiertes Aerosol erzeugt und durch ein elektrisches Hochspannungsfeld hindurchgeleitet. Dies so erzeugte Aerosol wird dem Patienten verabreicht.

Aufgabe der Erfindung ist, eine Vorrichtung der eingangs genannten Art so auszugestalten, dass deren therapeutische Wirksamkeit deutlich erhöht wird.

Erreicht wird dies dadurch, dass bei der Vorrichtung der eingangs genannten Art ein UV-Brenner zur Anhebung des Anteils an molekularem, ein- und dreiatomigem Sauerstoff im Aerosol im Ausgabebereich des Aerosols angeordnet ist und im Bereich des UV-Brenners auf das erzeugte Aerosol einwirkende Elektroden angeordnet sind, die ihrerseits an eine hinsichtlich der Polarität, der Intensität und des zeitlichen Verhaltens steuerbare Gleichstromquelle angeschlossen sind.

Mit Hilfe einer solchen Vorrichtung kann erreicht werden, dass festgesetzte Sekrete in den Bronchien besser gelöst werden und darüber hinaus die Vitalisierung des Patienten durch erhöhte Sauerstoffzufuhr mit der Medikamentengabe einhergeht. Dass durch die verbesserte Vitalisierung des Patienten auch eine noch stärkere therapeutische Unterstützung stattfindet, ist eine bekannte Tatsache.

Für den Anwendungszweck in Frage kommende UV-Brenner sind im Handel erhältlich, wobei es nicht einmal auf die Ausführungsform selbst, beispielsweise ringförmig oder rechteckige Ausgestaltung, ankommt. In jedem Falle kann erreicht werden, dass sich im Aerosol einatomiger Sauerstoff befindet, der direkt auf das Sekret in den Bronchien einwirkt. Der molekulare Sauerstoff im Aerosol bewirkt den erforderlichen Gasaustausch im Körper des Patienten, während der dreiatomige Sauerstoff die aus der Ozontherapie bekannten Wirkungen entfaltet.

Die Einstellung des Wellenlängenspektrums des UV-Brenners geschieht im Hinblick auf die verwendeten Medikamente, die Verträglichkeit und die therapeutische Wirksamkeit, da nämlich eine Ionisierung von Medikamentenmolekülen vermieden werden muss. Es handelt sich hierbei jedoch um relativ einfach durchzuführende Versuche, die zu dem Ergebnis führen, dass je nach Eigenart des Medikamentes und der zugehörigen Wellenlänge eine Beziehung besteht, die zur optimalen Wirkung des Medikamentes führt.

Die therapeutische Wirkung der Vorrichtung gemäß der Erfindung wird noch weiter dadurch verstärkt, dass im Bereich des UV-Brenners, auf das erzeugte Aerosol einwirkende Elektroden angeordnet sind, die ihrerseits an eine hinsichtlich Polarität, Intensität und zeitlichem Verhalten steuerbare Gleichstromquelle angeschlossen sind. Mit Hilfe eines elektrischen Feldes kann zusätzlich noch der Anteil positiv und negativ geladener Ionen im Aerosol eingeregelt werden, und zwar so, dass hier eine weiterhin verstärkende therapeutische Wirkung erreicht wird.

Die Elektroden können je nach Wunsch gepolt werden, wobei im Rahmen der Erfindung auch daran gedacht ist, die Polarität in Abhängigkeit von der Zeit zu verändern.

## Patentansprüche

1. Vorrichtung mit einem Ultraschallvernebler, der ein Sauerstoff mitführendes Aerosol für therapeutische Zwecke erzeugt, welches entweder aus reinem Wasser, einem Wassergemisch mit mindestens einem therapeutischen Wirkstoff oder lediglich aus einem therapeutischen Wirkstoff gebildet wird, **dadurch gekennzeichnet, dass** ein UV-Brenner zur Anhebung des Anteils an molekularem, ein- und dreiatomigem Sauerstoff im Aerosol im Ausgabebereich des Aerosols angeordnet ist und im Bereich des UV-Brenners auf das erzeugte Aerosol einwirkende Elektroden angeordnet sind, die ihrerseits an eine hinsichtlich der Polarität, der Intensität und des zeitlichen Verhaltens steuerbare Gleichstromquelle angeschlossen sind.

## Claims

1. Device comprising an ultrasonic atomizer, which produces aerosol entraining oxygen for therapeutic purposes, which is formed either from pure water, a water mixture with at least one therapeutic agent or only a therapeutic agent, **characterised in that** an UV-burner for increasing the content of molecular, monoatomic and triatomic oxygen in the aerosol is arranged in the output area of the aerosol, and electrodes acting on the produced aerosol are arranged in the area of the UV-burner, which in turn are connected to a direct current source controllable regarding the polarity, the intensity and the time response.

## Revendications

1. Dispositif comprenant un nébuliseur à ultrasons qui produit un aérosol entraînant de l'oxygène à des fins thérapeutiques, lequel est composé d'eau pure, d'un mélange d'eau et d'au moins un principe actif sur le plan thérapeutique ou seulement d'un principe actif sur le plan thérapeutique, **caractérisé en ce qu'**une lampe UV est disposée dans la zone de délivrance de l'aérosol afin d'augmenter la teneur en oxygène moléculaire, monoatomique et triatomique dans l'aérosol, et **en ce que** des électrodes, agissant sur l'aérosol produit, sont disposées dans la zone de la lampe UV, et sont, quant à elles, raccordées à une source de courant continu, qui est réglable quant à la polarité, l'intensité et le comportement dans le temps.
